# EUROPÄISCHE PATENTSCHRIFT

(11) **EP 0 402 584 B1**
(45) Veröffentlichungstag und Bekanntmachung des Hinweises auf die Patenterteilung: **02.11.1994**
(21) Anmeldenummer: 90106214.1
(22) Anmeldetag: 31.03.1990
(51) Int. Cl.: A61B 17/22, A61B 6/12

(54) **Zieleinrichtung für Lithotripter**
Aiming device for lithotriptor
Dispositif de visée pour lithotripteur

(30) Priorität: 10.06.1989 DE 3919083
(43) Veröffentlichungstag der Anmeldung: 19.12.1990
(73) Patentinhaber: DORNIER MEDIZINTECHNIK GMBH, D-82101 Germering (DE)
(72) Erfinder: Einars, Wolfram, Dr., D-8011 Neukeferloh (DE); Böhm, Klaus, D-8000 München 60 (DE); Herrmann, Bernhard, D-8034 Germering (DE); Neumann, Jürgen, D-8031 Alling (DE)
(74) Vertreter: Landsmann, Ralf, Dipl.-Ing.

(56) Entgegenhaltungen:
- EP-A- 0 018 166
- EP-A- 0 257 429
- US-A- 3 711 712
- US-A- 4 764 944

## Beschreibung

Die Erfindung betrifft eine Zieleinrichtung zur Röntgenortung bei der extrakorporalen Behandlung vom Patienten mit fokussierten Stosswellen, insbesondere für einen Lithotripter.

Die nicht invasive Steinzerkleinerung mit fokussierten Stosswellen erfordert eine genaue Ortung des Steines und eine genaue Positionierung des Patienten gegenüber dem Fokuspunkt, in dem sich die Stosswellen treffen.

Angewandt werden bisher die Röntgenortung und die Ortung mit Ultraschallgeräten. Während die Ultraschallortung kontinuierlich durchführbar ist, scheidet ein permanentes "Röntgen-Filmen" wegen der Strahlenbelastung aus. Auf der anderen Seite lassen sich nicht alle Steinsorten vom Ultraschallgerät ausreichend abbilden.

Eine Methode und ein Gerät zur Lokalisierung eines Fremdkörpers in einem Patientenauge mittels gekreuzter Röntgenaufnahmen beschreibt die Schrift US-A-3,711,712, wobei eine genaue Lokalisierung allerdings allein in Bezug auf den Patientenkörper möglich ist.

Die Erfindung geht aus von einer Zieleinrichtung gemäß EP-A-0 257 429, welche eine Einrichtung gemäß dem Oberbegriff des Anspruchs zum Inhalt hat.

Es ist Aufgabe der Erfindung, eine Zieleinrichtung zu schaffen, die eine einfache und genaue Ortung des zu behandelnden Objektes in einem Patientenkörper in Relation zum Therapiefokus direkt bei der Röntgenaufnahme ermöglicht, ohne daß der Patient einer erhöhten Strahlenbelastung durch wiederholte oder kontinuierliche Aufnahmen ausgesetzt ist.

Diese Aufgabe wird von einer Zieleinrichtung mit den Merkmalen des Anspruchs 1 gelöst. Ausführungen der Erfindung sind Gegenstände von abhängigen Ansprüchen.

Mit der erfindinngsgemäßen Zieleinrichtung kann eine Positionierung mit Hilfe eines Röntgen-C-Bogens, der eine Röntgenröhre und ihr gegenüber einen Bildverstärker enthält, mit wenigen Bildern - d.h. mit geringer Strahlenbelastung - erreicht werden. Die Zieleinrichtung enthält auf einem röntgennegativen Material mindestens vier röntgenpositive Referenzpunkte, von denen mindestens zwei auf einer Geraden liegen, die durch den Stosswellenfokus geht, und mindestens zwei weitere Referenzpunkte, die auf einer anderen Geraden, die ebenfalls durch den Fokuspunkt verläuft, liegen. Mit Hilfe dieser vier Punkte läßt sich aus zwei Positionen des Röntgen-C-Bogens der Steinort finden und positionieren. Die Auswertung erfolgt nach dem "Kimme und Korn" Prinzip und kann auch von Hilfskräften am Monitor durchgeführt werden.

Der Winkel zwischen den beiden Geraden, die in etwa den Visierlinien entsprechen, liegt bevorzugt zwischen 15 und 45°, insbesondere im Bereich von 30°. Der Abstand zweier Referenzpunkte auf einer Geraden liegt im Bereich zwischen 5 und 20 cm, insbesondere bei 10 cm.

Für den Träger kommt ein röntgennegatives Material, wie z.B. der Kunststoff Delrin (eingetragenes Warenzeichen), in Betracht. Der Träger soll möglichst nicht auf dem Bildschirm sichtbar werden. Die Massenverteilung senkrecht zur Durchstrahlungsrichtung sollte möglichst homogen sein. Eine Hohlkörperkonstruktion ist ebenso möglich. Die Außenkontur sollte auf der dem Patienten zugewandten Seite körperfreundlich sein, d.h. keine scharfen Kanten aufweisen. Der Träger sollte möglichst einfach an der Stosswellenqueele befestigbar und wieder abnehmbar sein und so stabil, daß sich die Zieleinrichtung unter einer Last von ca. 30 kg nur soweit verformt, daß eine Verschiebung der Zielgeraden im mm-Bereich bleibt. Eine solche Belastung kann durch Anstossen des Zielgeräts an den Patientenkörper erfolgen.

Die Referenzpunkte sollten aus möglichst röntgenpositivem Material bestehen, das sich kontrastreich auf dem Bildschirm darstellt. Als Material kommen alle Metalle mit hoher Ordnungszahl in betracht, wie zum Beispiel Stahl, Blei oder Lötzinn mit hohem Bleianteil.

Die Form der Referenzpunkte ist beliebig wählbar. Im einfachsten Fall können dies zwei Kugeln sein. Möglich sind auch Ausführungen wie Kimme und Korn, ein Quadrat, ein Dreieck, ein Kreis oder irgend eine andere geometrische Figur, die mit Fadenkreuz oder ohne Fadenkreuz ausgeführt sein können. Günstig ist es, wenn sich auf einer Visierlinie jeweils zwei unterschiedliche geometrische Figuren (z.B. ein Quadrat und ein Dreieck) befinden.

Zur nochmaligen Reduzierung der Strahlenbelastung beim Einrichten des Röntgen-C-Bogens kann auch eine Lochblende an der Zieleinrichtung vorgesehen sein, die in beiden Positionen fixierbar ist. Möglich sind ebenso zwei Blenden. In einer Ausführungsform beträgt der freie Blendendurchmesser 20 mm, die Blenden sind konzentrisch zu den Zielgeraden ausgerichtet, die Aussendurchmesser sind grösser 10 cm, als Blendenmaterial könnte 3 mm dickes Blei verwendet werden. Die Blende ist bevorzugt wegklappbar oder einfach abnehmbar ausgebildet.

Die Erfindung wird anhand von drei Figuren näher erläutert.

Es zeigen:
- Figur 1: eine erfindungsgemässe Zieleinrichtung,
- Figur 2: Ausführungen von Referenzpunkten und
- Figur 3: ein Auswerteverfahren anhand zweier Bildschirmdarstellungen.

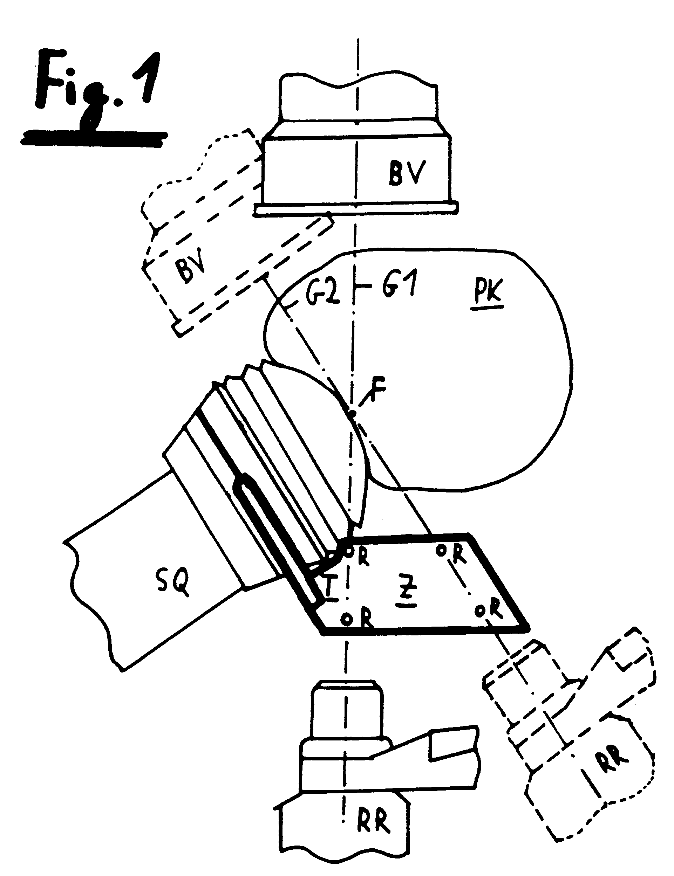

Figur 1 zeigt einen Patientenkörper, der innerhalb eines Röntgen-C-Bogens auf einer nicht gezeigten Liege angeordnet ist. Der Röntgen-C-Bogen enthält unten eine Röntgenröhre RR und oben einen Bildverstärker BV, die kolinear angeordnet sind. Der Röntgen-C-Bogen ist um eine Achse, die ungefähr parallel zur Längsachse des Patientenkörpers liegt, verschwenkbar, so dass die in Figur 1 gestrichelte Position von Röntgenröhre RR und Bildverstärker BV erreichbar ist. Am Patientenkörper PK befindet sich die Stosswellenquelle SQ, die hier an ihrer Vorderseite mit einem elastischen, flexiblen Balg abgeschlossen ist. Die Stosswellenquelle SQ erzeugt Stosswellen, fokussiert diese und leitet sie zum Fokuspunkt F. Die erfindungsgemässe Zieleinrichtung Z enthält einen Träger T, mittels dessen sie an der Stosswellenquelle SQ befestigt werden kann und in dieser Ausführung vier Referenzpunkte R. Die beiden linken Referenzpunkte R liegen auf der Geraden G1, die durch den Fokuspunkt F läuft, die beiden rechten Referenzpunkte R liegen auf der Geraden G2, die ebenfalls durch den Fokuspunkt F läuft.

Zur Röntgenortung wird nun wie folgt vorgegangen:
Die Stosswellenquelle SQ wird etwa in die Behandlungsstellung gebracht und die Zieleinrichtung Z angeklemmt. Die Liege mit dem Patientenkörper PK wird eingeschwenkt und nach entsprechender Lagerung des Patienten wird die Stosswellenquelle angekoppelt. Der Röntgen-C-Bogen wird frei beigestellt. Auf dem Boden des Behandlungsraumes können zum Beispiel Markierungen aufgetragen sein, auf die er positioniert werden soll. Die Strahlrichtung ist durch die Zieleinrichtung, durch die beiden linken Referenzpunkte R, grob vorgegeben. In diese Gerade wird der Zentralstrahl ungefähr ausgerichtet (durchgezogen gezeichnete Geräte RR und BV).

Beim Aufnehmen eines Röntgenfotos sind nun die beiden Referenzpunkt R aufeinander oder nebeneinander vollständig auf dem Bildschirm dargestellt. Aus der Lage der Referenzpunkte im Bild und der entsprechenden Abbildungsvergrösserung ist die Lage des Strahlbüschels zur Stosswellenquelle definiert. Aus der bekannten Stellung der Stosswellenquelle ist somit die Lage und Richtung der Röntgenstrahlung bezüglich des Referenzsystems errechenbar. Auf dem Bildschirm ist auch der Stein S sichtbar. Der Patientenkörper PK wird nun so verschoben, dass der Stein S mit dem Fokus F zur Deckung kommt. Dies ist durch Kontrollaufnahmen nachprüfbar. Der Stein liegt jetzt auf einer Geraden, die durch den Fokuspunkt F geht.

Im zweiten Schritt wird der Röntgen-C-Bogen um einen festen Winkel (z.B. 30°) gekippt. Die Visierlinie des Röntgengeräts entspricht in etwa der Linie, die von den beiden rechten Referenzpunkten R definiert wird. Bei einer Aufnahme zeigt das Bild nun wieder die beiden Referenzpunkte R und den Stein. Aus der Lage der Referenzpunkte R kann auf den Fokus geschlossen werden. Der Stein kann durch nochmaliges Verschieben des Patientenkörpers PK auf den Fokus F positioniert werden. Durch weitere Aufnahmen kann der Erfolg der Positionierung kontrolliert werden. Da der Stein in der zweiten Projektion auf einer bekannten Geraden, die durch F führt, liegt, kann aus dieser Aufnahme der Betrag errechnet werden, um den der Stein (der Patient) auf dieser Geraden bewegt werden muß.

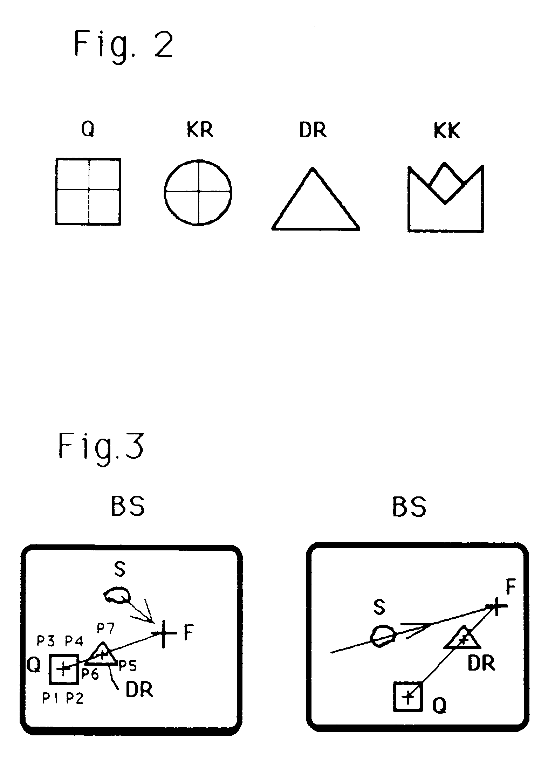

Figur 2 zeigt vier mögliche Ausführungen von Referenzpunkten, nämlich ein Quadrat Q, einen Kreis KR, ein Dreieck DR oder eine Ausführung mit Kimme und Korn KK. In dem Quadrat Q und im Kreis KR ist jeweils ein Fadenkreuz aus röntenpositivem Material eingebracht. Durchführbar sind die Positionierungen aber auch mit Figuren (Referenzpunkten) ohne Fadenkreuz.

Figur 3 zeigt zwei Bildschirme, anhand derer die Ortung nochmals erklärt werden kann. Der linke Bildschirm BS zeigt eine Aufnahme aus einer ersten Position, bei der Röntgenröhre und Bildverstärker in der Nähe der in Figur 1 durchgezogen gezeichneten Position stehen. Als Referenzpunkte R sind hier ein Quadrat Q und ein Dreieck DR vorgesehen. Das Bild zeigt die beiden Körper, das Quadrat Q und das Dreieck DR. Aus deren Lage, insbesondere aus der gegenseitigen Lage der Schwerpunkte der Figuren lässt sich der Brennpunkt F berechnen. Dieser liegt auf einer Geraden, die durch die beiden Schwerpunkte der Figuren Q und DR führt. Auf dem Bild ist auch der Stein S zu sehen. Der behandelnde Arzt kann nun den Patienten so verschieben, dass der Stein S mit dem Brennpunkt F zusammenfällt. Eine mögliche Auswertung kann zum Beispiel dadurch erfolgen, dass ein Light pen vorgesehen ist, mit dem der Arzt die Eckpunkte des Quadrates P1, P2, P3 und P4 sowie die Eckpunkte des Dreiecks P5, P6 und P7 antippt. Ein Rechner kann aus diesen sieben Koordinaten die Schwerpunkte bilden, eine Gerade durch diese Punkte im Bildschirm erzeugen und die Lage des Brennpunkts F generieren. Auch das Verfahren des Tisches kann automatisch erfolgen, indem der Arzt nach Antippen der Figuren Q und DR auch den Stein S antippt. Eine Routine kann den Tisch dann entsprechend zu dem fiktiven Ort des Fokuspunktes auf dem Monitor verfahren.

Figur 3 zeigt rechts auf dem Bildschirm BS eine Aufnahme, die aus der anschliessenden zweiten Position des Röntgen-C-Bogens (in der Nähe der in Figur 1 gestrichelt gezeigten Position) aufgenommen wurde. Zu erkennen sind wieder die Figuren Q und DR, sowie der Stein S. Aus den beiden Figuren Q und DR kann der Rechner wiederum den Brennpunkt F berechnen. Da der Stein S bereits auf einer definierten Geraden durch den Fokus F liegt, kann auch jetzt die Verschiebung des Steins S in den Fokuspunkt F automatisch erfolgen.

Eine andere Möglichkeit der Auswertung, bei der auf die Eingabe der Punkte P1 bis P7 verzichtet werden kann, ist das Aufrufen einer Bilderkennungsroutine, welche die Figuren Q und DR erkennt, aus der gegenseitigen Lage und der Grösse der Figuren den Abbildungsmaßstab und die Lage des Brennpunkts F berechnen kann.

## Patentansprüche

1. Zieleinrichtung (Z) zur Röntgenortung bei einer Vorrichtung zur extrakorporalen Behandlung von Patienten mit fokussierten Stosswellen, insbesondere für einen Lithotripter, **gekennzeichnet** durch einen an der Stosswellenquelle (SQ) befestigbaren Träger (T) aus röntgennegativem Material, der mindestens vier röntgenpositive Referenzpunkte (R) trägt, von denen mindestens zwei auf einer ersten Geraden (G1) liegen, die durch den Stosswellenfokus (F) führt, und von denen mindestens zwei weitere auf einer zweiten Geraden (G2) liegen, die ebenfalls durch den Stosswellenfokus (F) führt.

2. Zieleinrichtung (Z) nach Anspruch 1, **dadurch gekennzeichnet,** daß die beiden Geraden (G1, G2) einen Winkel zwischen 15° und 45°, insbesondere um 30°, einschließen.

3. Zieleinrichtung (Z) nach Anspruch 1 oder 2, **dadurch gekennzeichnet,** daß der Abstand zweier Referenzpunkte (R) auf beiden Geraden (G1, G2) im Bereich von 5 bis 20 cm, insbesondere bei 10 cm liegt.

4. Zieleinrichtung (Z) nach einem der vorhergehenden Ansprüche, **dadurch gekennzeichnet,** daß die Referenzpunkte (R) als geometrische Figuren wie Kugeln oder Kimme und Korn (KK), Quadrat (Q), Dreieck (DR) oder Kreis (KR), jeweils mit oder ohne Fadenkreuz, ausgebildet sind und auf jeder der genannten Geraden (G1, G2) jeweils zwei verschiedene Figuren angeordnet sind.

## Claims

1. Aiming device (Z) for X-ray positioning in a device for extra-corporal treatment of patients with focussed shockwaves, in particular a lithotripter, **characterised by** a carrier (T) of X-ray negative material, which is attached to the shockwave source (SQ) and with at least four X-ray positive reference points (R) of which at least two are on a first plane (G1) which extends through the shockwave focus (F), and of which at least an additional two are on a second plane (G2) which also extends through the shock-wave focus F).

2. Aiming device (Z) according to claim 1, **characterised in that** both planes (G1, G2) include an angle of between 15° and 45°, in particular around 30°.

3. Aiming device (Z) according to claim 1 or 2, **characterised in that** the distance between both reference points (R) on both planes (G1, G2) is in the region of between 5 and 20 cm, in particular at 10 cm.

4. Aiming device (Z) according to one of the above claims, **characterised in that** the reference points (R) are arranged as geometrical figures, such as balls or bead and notch (KK), square (Q), triangle (DR) or circle (KR), with or without respective graticule, and that on each of the aforementioned planes (G1, G2) are arranged two respective different figures.

## Revendications

1. Dispositif monté sur un lithotriteur pour le repérage de concrétions dans le corps d'un patient, le dispositif comportant un transducteur à ultrasons (TR) qui est guidé de telle sorte que son axe médian soit dirigé sur un point déterminé (isocentre), la tête de traitement (TK) comportant des moyens pour produire, focaliser et introduire des ondes de choc dans le corps (PK) du patient et l'isocentre du transducteur (TR) coïncidant avec le foyer de traitement (F) de la tête de traitement (TK), caractérisé par le fait que le transducteur (TR) est fixé sur la tête de traitement mobile au moyen d'un ensemble cinématique qui comprend trois leviers (3a, 3b, 3c), le premier levier (3a) et le troisième levier (3c) étant guidés de manière telle que ceux-ci soient toujours parallèles entre eux.

2. Dispositif selon la revendication 1, caractérisé par le fait que le transducteur (TR) est fixé sur un anneau (R) qui entoure la tête de traitement (TK) et dont l'axe de rotation coïncide avec l'axe de ladite tête de traitement (TK)

3. Dispositif selon l'une des revendications précédentes, caractérisé par le fait que le transducteur (TR) est mobile dans la direction de son axe longiudinal.

4. Dispositif selon l'une des revendications précédentes, caractérisé par le fait que le transducteur (TR) est mobile en rotation autour de son axe longitudinal.
